# FASCICULE DE BREVET EUROPEEN

(11) **EP 4 209 243 B1**
(45) Date de publication et mention de la délivrance du brevet: **14.08.2024**
(21) Numéro de dépôt: 22210985.2
(22) Date de dépôt: 02.12.2022
(51) Int. Cl.: A61M 16/12, A61M 16/00, A61M 16/20

(54) **DISPOSITIF DE DÉLIVRANCE DE NO AVEC SYSTÈME DE DOSAGE D'URGENCE**
NO-AUSGABEVORRICHTUNG MIT NOTFALLDOSIERSYSTEM
NO DELIVERY DEVICE WITH EMERGENCY DOSING SYSTEM

(30) Priorité: 05.01.2022 FR 2200064; 18.07.2022 FR 2207324
(43) Date de publication de la demande: 12.07.2023
(73) Titulaire: Inosystems, 92160 Antony (FR)
(72) Inventeur: BOULANGER, Thierry, 92160 Antony (FR); MARCHAL, Frédéric, 92160 Antony (FR); SCHMITT, Mary, 92220 Bagneux (FR)
(74) Mandataire: Air Liquide

(56) Documents cités:
- EP-A1- 2 522 384
- EP-A1- 3 888 727
- WO-A1-2016/096056

## Description

L'invention concerne un dispositif de délivrance de monoxyde d'azote gazeux (NO) à un patient comprenant un système de dosage d'urgence de NO, et destiné à être connecté au circuit patient d'un ventilateur mécanique, c'est-à-dire un appareil médical d'administration de gaz à un patient, lequel permet de fournir le gaz à débit préfixé en cas de dysfonctionnement, notamment des moyens de pilotage.

Le NO est un gaz qui, lorsqu'inhalé, dilate les vaisseaux pulmonaires et augmente l'oxygénation en améliorant les échanges gazeux.

Les propriétés du NO sont utilisées pour traiter différentes conditions médicales, comme l'Hypertension Artérielle Pulmonaire du nouveau-né ou PPHN (pour *Persistent Pulmonary Hypertension of the Newborn*), le Syndrome de Détresse Respiratoire Aigüe ou SDRA observé principalement chez l'adulte ou encore les hypertensions pulmonaires en chirurgie cardiaque, comme enseigné notamment par EP-A-560928, EP-A-1516639 ou US-A-10,201,564.

Usuellement, une faible quantité de NO gazeux (i.e. quelques ppm vol.), dilué dans de l'azote (N₂) est injecté dans un flux gazeux contenant de l'oxygène (O₂) qui est ensuite inhalé par le patient. La concentration de NO, qui correspond à une posologie, est déterminée par le médecin ou analogue. Typiquement, le gaz contenant I'O₂ est typiquement un mélange N₂/O₂ ou de l'air, tel de l'air de qualité médical. En général, la concentration de NO dans le gaz inhalé par le patient est comprise entre 1 et 80 ppm en volume (ppmv), en fonction de la population traitée, i.e. nouveau-nés ou adultes, et donc de la maladie à traiter.

Le gaz inhalé par le patient peut être délivré par le biais d'un dispositif de délivrance de NO associé à un ventilateur mécanique, comme décrit par US-A-5,558,083. Le dispositif de délivrance de NO est fluidiquement connecté à une ou plusieurs bouteilles de gaz contenant un mélange de N₂/NO dont la concentration en NO peut être comprise typiquement entre 200 et 800 ppmv. Généralement, le système de délivrance de NO comprend un module d'injection de NO placé dans la branche inspiratoire d'un circuit patient connecté fluidiquement, d'une part, au ventilateur mécanique et, d'autre part, à une interface respiratoire délivrant le gaz enrichi en NO au patient, par exemple un masque respiratoire, une sonde d'intubation trachéale ou similaire.

Le système de délivrance de NO comprend également un capteur de débit qui mesure le débit gazeux délivré par le ventilateur mécanique (i.e. air ou mélange N₂/O₂) afin de déterminer la quantité de NO à délivrer pour respecter la posologie fixée par le médecin.

Le système de délivrance de NO peut assurer le dosage en NO par le biais d'une électrovanne proportionnelle délivrant un flux continu de gaz contenant le NO, laquelle est associée à un capteur de débit, les deux composants étant agencés dans le système de délivrance, ainsi qu'une ligne d'injection reliée au module d'injection de NO, tel que décrit dans US-A-5,558,083.

D'autres systèmes sont disponibles où l'électrovanne proportionnelle est remplacée par une pluralité d'électrovannes de type « tout ou rien », délivrant le gaz de façon intermittente, c'est-à-dire sous forme de pulses, généralement à haute fréquence, dont l'amplitude et la durée permettent de garantir la bonne quantité de gaz circulant dans la ligne d'injection reliée au module d'injection de NO.

Par exemple, on connait les documents EP2522384, EP3888727 et WO2016/096056 qui décrivent des appareils de fourniture de NO d'architecture classique comprenant notamment une ligne d'injection de NO pour acheminer le gaz contenant du NO, un dispositif à vanne agencé sur la ligne d'injection pour contrôler la circulation du gaz contenant du NO dans la ligne d'injection, un dispositif de mesure de débit agencé sur la ligne d'injection pour opérer une ou des mesures de débit du gaz contenant du NO circulant dans la ligne d'injection, et des moyens de pilotage.

Dans tous les cas, les systèmes de délivrance de NO connus reçoivent les mesures du capteur de débit placé dans la branche inspiratoire du circuit patient et ajustent en temps réel la quantité de NO devant être délivrée, selon la posologie désirée, en contrôlant le flux de NO dans la ligne d'injection.

Le NO étant un agent thérapeutique efficace, c'est-à-dire que de très faibles concentrations (i.e. quelques ppmv) produisent un effet thérapeutique, son juste dosage est d'importance critique et les équipes médicales doivent constamment adapter la posologie selon l'état du patient.

Lorsque l'état du patient évolue, la concentration de NO doit être progressivement diminuée ou augmentée. Par exemple, dans une situation de sevrage du nouveau-né dont l'état s'améliore, il est usuel de décroitre progressivement la posologie, par exemple par pas de 1 ppm, jusqu'à atteindre une valeur nulle permettant alors de stopper le système de délivrance de NO.

Une diminution progressive de la concentration en NO permet d'éviter l'« effet rebond », pouvant se manifester en cas de variation rapide de la concentration, par exemple en cas de discontinuation brutale du traitement, avec pour effet d'empirer gravement l'état du patient.

Or, les systèmes de délivrance de NO sont des systèmes électro-médicaux sophistiqués susceptibles de subir des défaillances ou dysfonctionnements pouvant avoir un impact important sur la thérapie en cours. Par exemple, un dysfonctionnement ou défaut électronique majeur, en particulier des moyens de pilotage, peut entraîner une panne de l'appareil et donc un arrêt total de délivrance de NO, avec les conséquences négatives susmentionnées.

Dans de telles circonstances, le dispositif doit avertir l'utilisateur au moyen d'un signal d'alarme audible qu'une action rapide est requise, par exemple de basculer sur un mode d'injection pneumatique de secours afin de limiter autant que possible, les effets indésirables liés à une discontinuation de la thérapie.

Un tel basculement en mode de secours se fait habituellement par actionnement d'un bouton rotatif commandant le passage du mode d'administration normal de NO vers un mode de secours, dans lequel est par exemple opérée une délivrance continue d'un débit fixe de mélange N₂/NO, i.e. de l'ordre de 250 mL/min.

Or, un mécanisme ou système de dosage de secours n'est pas sans risque, notamment pour les raisons suivantes :
- son activation requiert la présence d'une personne ayant autorité pour entreprendre cette action, par exemple un médecin en néonatologie. Il peut ainsi se passer plusieurs minutes avant que cette personne arrive et donc que le dosage de secours ne soit établi, ce qui entraine une discontinuation de la thérapie et expose le patient à un effet rebond.
- ce dosage de secours, i.e. un débit unique de mélange N₂/NO, ne permet pas de garantir que la posologie désirée soit respectée. En particulier, quand le dosage de secours est très inférieur à la posologie souhaitée, le patient peut être exposé à un changement abrupte de concentration et potentiellement sujet à d'importants effets indésirables.
- le dosage de secours est incompatible avec certains types de ventilateurs délivrant de très faibles volumes, tels que les ventilateurs à oscillations à haute fréquence (HFO pour *High Frequency Oscillations*) car il peut en résulter une concentration en NO inhalée trop élevée pouvant atteindre des niveaux dangereux pour le patient. Dès lors, en cas de traitement du patient avec un tel ventilateur (i.e. HFO), on se retrouve alors sans moyens d'administrer le NO au patient, ce qui entraine les risques susmentionnés liés à l'arrêt brusque de traitement.

Il apparaît dès lors que les mécanismes de dosage de secours actuels ne permettant pas de garantir un niveau satisfaisant de sécurité et qu'il serait souhaitable pour le patient, en cas de mise en place d'un dosage de secours du fait d'un dysfonctionnement du système de délivrance de NO, de pouvoir maintenir une thérapie par NO, sans commettre d'interruption de la thérapie, et sans se soucier du type de ventilateur, i.e. avec fonction HFO ou autre, avec lequel coopère le système de délivrance de NO.

Autrement dit, un problème est de pouvoir maintenir une posologie, c'est-à-dire un traitement du patient par NO inhalé, même en cas de défaillance ou dysfonctionnement du système de délivrance de NO, en particulier un arrêt total de fonctionnement des moyens de pilotage du fait d'une panne ou d'un défaut d'alimentation électrique par exemple.

Une solution selon l'invention concerne un dispositif, i.e. un appareil, de délivrance de NO pour fournir un gaz contenant du NO, typiquement un mélange NO/azote, comprenant :
- une ligne d'injection de NO pour acheminer le gaz contenant du NO,
- un dispositif à vanne agencé sur la ligne d'injection pour contrôler la circulation du gaz contenant du NO dans la ligne d'injection, ledit dispositif à vanne étant configuré pour être normalement dans une position fermée pour empêcher toute circulation de gaz dans la ligne d'injection,
- un dispositif de mesure de débit agencé sur la ligne d'injection pour opérer une ou des mesures de débit du gaz contenant du NO circulant dans la ligne d'injection,
- une ligne de secours venant se raccorder fluidiquement à la ligne d'injection en amont et en aval du dispositif à vanne, ladite ligne de secours comprenant une électrovanne de secours configurée pour être normalement dans une position ouverte pour permettre une circulation de gaz dans la ligne de secours, et un dispositif de contrôle de débit formant ou comprenant un système à orifice calibré proportionnel, et
- des moyens de pilotage, aussi appelés unité de pilotage, configurés pour coopérer avec l'électrovanne de secours, le dispositif de contrôle de débit, le dispositif à vanne et le dispositif de mesure de débit.

De plus, dans le dispositif de l'invention, en cas de dysfonctionnement (i.e. au sein du dispositif de délivrance de NO) engendrant un arrêt de toute coopération avec les moyens de pilotage :
- l'électrovanne de secours est configurée pour passer en position ouverte pour permettre une circulation de gaz dans la ligne de secours,
- le dispositif à vanne est configuré pour passer en position fermée pour stopper toute circulation de gaz dans la ligne d'injection, et
- le dispositif de contrôle de débit est configuré pour fournir le gaz à un débit gazeux de secours préfixé, où ledit débit gazeux de secours :
   o est déterminé par les moyens de pilotage à partir d'au moins une mesure de débit du gaz fournie par le dispositif de mesure de débit, pendant un fonctionnement normal du dispositif précédant ledit dysfonctionnement, et
   o est préréglé par commande dudit dispositif de contrôle de débit par les moyens de pilotage, pendant ledit fonctionnement normal du dispositif.

Dans le cadre de l'invention :
- « ppmv » signifie partie par million en volume,
- « %vol. » » signifie pourcentage en volume.
- « NO » désigne le monoxyde d'azote.
- « N₂ » désigne l'azote.
- « O₂ » désigne l'oxygène.
- Par « fonctionnement normal » : on entend un fonctionnement habituel du dispositif de délivrance de NO pendant une première période de temps (de durée non-nulle), en l'absence de toute panne, dysfonctionnement, défaut ou autre. La première période de temps a une durée de typiquement une à plusieurs minutes, voire heures ou même plus.
- Par « dysfonctionnement », on entend une panne, une anomalie, un problème, un malfonctionnement, un défaut ou analogue, qu'il soit électrique, mécanique ou d'une autre nature, affectant le fonctionnement normal du dispositif de délivrance de NO, en particulier empêchant le fonctionnement des moyens de pilotage du dispositif, pendant une seconde période de temps (de durée non-nulle), par exemple du fait d'une panne des moyens de pilotage, typiquement un défaut d'alimentation électrique de ceux-ci. La seconde période de temps a une durée variable, par exemple de quelques secondes à une ou plusieurs minutes, ou dizaine(s) de minutes, ou même plus.

Selon le mode de réalisation considéré, le dispositif de délivrance de NO de l'invention peut comprendre l'une ou plusieurs des caractéristiques suivantes :
- les moyens de pilotage sont en outre configurés pour, pendant le fonctionnement normal du dispositif, commander l'électrovanne de secours pour qu'elle soit en une position fermée empêchant toute circulation de gaz dans la ligne de secours.
- les moyens de pilotage sont en outre configurés pour, pendant le fonctionnement normal du dispositif, commander le dispositif à vanne pour autoriser une circulation de gaz dans la ligne d'injection et permettre d'opérer au moins une mesure de débit de gaz par le dispositif de mesure de débit.
- les moyens de pilotage sont en outre configurés pour, pendant le fonctionnement normal du dispositif, contrôler le dispositif de contrôle de débit pour prérégler le débit gazeux de secours à partir d'au moins une mesure de débit du gaz fournie par le dispositif de mesure de débit. Autrement dit, le réglage du dispositif de contrôle de débit se fait préalablement à tout dysfonctionnement, c'est-à-dire pendant que le dispositif fonctionne normalement.
- pendant le fonctionnement normal du dispositif, le dispositif de mesure de débit est configuré pour opérer plusieurs mesures de débit successives.
- pendant le fonctionnement normal du dispositif, les moyens de pilotage sont en outre configurés pour déterminer, par exemple calculer, le débit gazeux de secours à partir d'une ou plusieurs mesures de débit opérées par le dispositif de mesure de débit.
- la ligne de secours vient se raccorder fluidiquement à la ligne d'injection en amont du dispositif à vanne, et en amont ou en aval du dispositif de mesure de débit, de préférence en aval du dispositif de mesure de débit.
- un dispositif de mesure de débit est agencé sur la ligne d'injection en amont ou en aval du dispositif à vanne, de préférence en aval du dispositif à vanne.
- la ligne de secours vient se raccorder fluidiquement à la ligne d'injection par une extrémité amont, en amont du dispositif à vanne et par une extrémité aval, en aval du dispositif à vanne de manière à bipasser ledit dispositif à vanne.
- il comprend des moyens de mémorisation pour mémoriser au moins une partie des mesures de débit successives opérées par le dispositif de mesure de débit, c'est-à-dire les mesures de débit successives sont mémorisées par des moyens de mémorisation
- les moyens de mémorisation comprennent une mémoire informatique, par exemple un mémoire vive, ou autre.
- il comprend un système de dosage de secours de NO comprenant la ligne de secours.
- la ligne d'acheminement de NO achemine un mélange gazeux formé de NO et d'azote, de préférence un mélange gazeux NO/N₂ (i.e. monoxyde d'azote/azote) contenant entre 100 et 2000 ppmv de NO, typiquement moins de 1000 ppmv de NO, le reste étant de l'azote (et éventuellement des impuretés inévitables)
- l'électrovanne de secours est configurée et/ou pilotée pour être normalement ouverte.
- l'électrovanne de secours est du type tout ou rien.
- les moyens de pilotage comprennent au moins un microprocesseur.
- les moyens de pilotage comprennent une carte électronique portant ledit au moins un microprocesseur.
- la ligne d'injection est raccordée fluidiquement à une ligne haute pression par l'intermédiaire d'un dispositif régulateur de pression, la ligne haute pression et le dispositif régulateur de pression étant agencés dans le dispositif de délivrance de NO.
- le dispositif de délivrance de NO comprend un boitier.
- le système de dosage de secours de NO est agencé dans le boitier, en particulier la ligne de secours et l'électrovanne de secours.
- la ligne de secours vient se raccorder fluidiquement à la ligne d'injection entre le dispositif régulateur de pression et le dispositif à vanne.
- le dispositif à vanne comprend une électrovanne, de préférence une électrovanne proportionnelle.
- le dispositif de contrôle de débit est configuré pour former, constituer ou comprendre un orifice calibré de type proportionnel, c'est-à-dire former un système à orifice calibré proportionnel.
- le dispositif de contrôle de débit comprend un moyen actionneur coopérant avec un élément mobile.
- le dispositif de contrôle de débit comprend un moyen actionneur coopérant avec un élément mobile comprenant un évidement traversant.
- l'élément mobile est configuré pour être déplacé angulairement par le moyen actionneur du dispositif de contrôle de débit.
- le moyen actionneur comprend un moteur électrique alimenté par les moyens d'alimentation électrique, i.e. pendant le fonctionnement normal.
- le moyen actionneur comprend un moteur électrique entraînant un axe rotatif, l'élément mobile étant solidaire dudit axe rotatif.
- le moteur électrique est un moteur pas à pas.
- l'élément mobile est agencé mobile dans un logement interne comprenant un port d'entrée et un port de sortie en communication fluidique avec la ligne de secours.
- l'élément mobile est une sphère, c'est-à-dire sphérique, par exemple une bille ou analogue.
- le logement interne a une forme sphérique complémentaire de celle de l'élément mobile sphérique.
- les moyens de pilotage sont configurés pour piloter le moyen actionneur pour opérer un déplacement angulaire de l'élément mobile entre au moins :
   o une position d'ouverture totale correspondant à un niveau d'ouverture totale de l'orifice calibré du dispositif de contrôle de débit,
   o une position de fermeture totale correspondant à un niveau de fermeture (i.e. obturation) totale de l'orifice calibré du dispositif de contrôle de débit, et
   o au moins une position intermédiaire située entre lesdites position d'ouverture totale et position de fermeture totale correspondant à un niveau d'ouverture partiel de l'orifice calibré du dispositif de contrôle de débit.
- les moyens de pilotage sont configurés pour piloter le moyen actionneur pour opérer un déplacement, de préférence angulaire, de l'élément mobile entre au moins :
   o une position d'ouverture totale dans laquelle tout le débit gazeux amené par la ligne de secours pénètre dans l'évidement traversant de l'élément mobile, c'est-à-dire un débit maximal,
   o une position de fermeture totale dans laquelle aucun débit gazeux ne peut traverser l'évidement traversant de l'élément mobile, c'est-à-dire un débit nul, et
   o au moins une position intermédiaire située entre lesdites position d'ouverture totale et position de fermeture totale, dans laquelle seulement une partie du débit gazeux amené par la ligne de secours pénètre dans l'évidement traversant de l'élément mobile, c'est-à-dire un ou des débits réduits.
- les moyens de pilotage sont configurés pour piloter le moyen actionneur pour opérer un déplacement, de préférence angulaire, de l'élément mobile entre plusieurs positions intermédiaires, angulairement décalées les uns des autres, correspondant chacune à un niveau d'ouverture d'orifice calibré et/ou à un débit de gaz donné, c'est-à-dire des débits réduits compris entre le débit maximal et le débit nul.
- les moyens de pilotage sont configurés pour piloter le moyen actionneur pendant le fonctionnement normal du dispositif, c'est-à-dire préalablement à tout dysfonctionnement, de manière à régler ou ajuster le débit gazeux de secours préfixé.
- les moyens de pilotage sont configurés pour piloter le moyen actionneur pour opérer un déplacement, de préférence angulaire, de l'élément mobile dans une position donnée correspondant au débit de secours préréglé.
- les moyens de pilotage sont configurés pour déterminer l'ouverture d'orifice calibré donné et/ou le débit de secours préréglé à partir d'une table de correspondance mémorisée.
- les moyens de pilotage sont configurés pour déterminer une ouverture d'orifice calibré donné correspondant au débit de secours préréglé.
- la table de correspondance est mémorisée par les moyens de mémorisation, telle une mémoire informatique.
- il comprend des moyens d'alimentation électrique configurés pour alimenter en courant électrique les composants nécessitant de l'énergie électrique pour fonctionner, notamment les moyens de pilotage ou d'autres composants, comme les électrovannes, le moteur électrique....
- les moyens d'alimentation électrique comprend des moyens de raccordement au secteur (110/220V) et/ou une batterie ou analogue.
- le dispositif de contrôle de débit du système de dosage de secours de NO constitue un système à orifice calibré proportionnel permettant de régler ou ajuster le débit gazeux de secours préfixé, préalablement à tout dysfonctionnement de l'appareil empêchant toute coopération entre les moyens de pilotage et l'électrovanne de secours, le dispositif de contrôle de débit, le dispositif à vanne et/ou le dispositif de mesure de débit.
- le débit gazeux de secours à partir correspond à la dernière mesure de débit opérée par le dispositif de mesure de débit ayant été opérée avant le dysfonctionnement.

Selon un mode de réalisation particulier, l'invention concerne aussi un dispositif de délivrance de NO pour fournir un gaz contenant du NO comprenant
- une ligne d'injection de NO pour acheminer le gaz contenant du NO,
- un dispositif à vanne agencé sur la ligne d'injection pour contrôler la circulation du gaz contenant du NO dans la ligne d'injection,
- un dispositif de mesure de débit agencé sur la ligne d'injection, en aval du dispositif à vanne,
- un système de dosage de secours de NO,
- des moyens de pilotage,
- et des moyens d'alimentation électrique alimentant en courant électrique au moins les moyens de pilotage,

dans lequel le système de dosage de secours de NO comprend une ligne de secours venant se raccorder fluidiquement à la ligne d'injection en amont du dispositif à vanne, ladite ligne de secours comprenant une électrovanne de secours et un dispositif de contrôle de débit formant un système à orifice calibré proportionnel, l'électrovanne de secours et le dispositif de contrôle de débit étant pilotés par les moyens de pilotage,
et dans lequel :
   - les moyens de pilotage sont configurés pour :
      a) calculer un débit de NO moyen à partir du débit de NO ayant circulé dans la ligne d'injection pendant un temps donné, c'est-à-dire pendant le fonctionnement normal du dispositif de délivrance de NO, et
      b) contrôler le dispositif de contrôle de débit pour prérégler un niveau d'ouverture donné de l'orifice calibré du dispositif de contrôle de débit, c'est-à-dire pendant le fonctionnement normal du dispositif de délivrance de NO,
      c) et, en cas dysfonctionnement, c'est-à-dire de défaillance majeure du dispositif de délivrance de NO ou d'interruption de l'alimentation électrique du dispositif de délivrance de NO,
         i) le dispositif à vanne est configuré pour passer en position fermée pour empêcher le passage du gaz, i.e. de mélange NO/N₂, dans la ligne d'injection, et
         ii) l'électrovanne de secours est configurée pour passer en position ouverte pour autoriser le passage du gaz, i.e. de mélange NO/N₂, dans la ligne de secours à un débit fixé par le niveau d'ouverture de l'orifice calibré correspondant à la dernière valeur de débit de NO moyen déterminée par l'unité de pilotage, c'est-à-dire la dernière valeur valide de débit de NO moyen ayant été déterminée avant dysfonctionnement, c'est-à-dire pendant le fonctionnement normal ayant précédé ce dysfonctionnement.

L'invention concerne aussi une installation de fourniture de gaz à un patient, c'est-à-dire un être humain, comprenant :
- au moins une source de NO contenant un mélange gazeux NO/N₂,
- un dispositif de délivrance de NO selon l'invention, alimenté en mélange gazeux NO/N₂ par ladite au moins une source de NO,
- une branche inspiratoire d'un circuit patient alimentée en mélange gazeux NO/N₂ par le dispositif de délivrance de NO, et
- un ventilateur médical, i.e. un appareil d'assistance respiratoire, en communication fluidique avec la branche inspiratoire pour alimenter ladite branche inspiratoire en un gaz respiratoire contenant au moins 21% d'oxygène.

Selon le mode de réalisation considéré, l'installation de fourniture de gaz de l'invention peut comprendre l'une ou plusieurs des caractéristiques suivantes :
- le ventilateur médical délivre de l'air ou un mélange oxygène/azote, i.e. en tant que gaz respiratoire contenant au moins 21% vol. d'oxygène.
- selon un mode de réalisation, le ventilateur médical comprend une soufflante motorisée (i.e. turbine, compresseur ou analogue) délivrant le gaz respiratoire, typiquement de l'air ou un mélange oxygène/azote.
- selon un autre mode de réalisation, le ventilateur médical comprend un circuit de gaz interne comprenant une ou des vannes proportionnelles pour acheminer le gaz et contrôler sa fourniture, notamment son débit. Un tel ventilateur est généralement alimentée en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens de commande, telle une (ou des) carte de commande électronique.
- les moyens de commande, telle une carte de commande électronique, pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- la source de NO contient un mélange gazeux NO/N₂ contenant entre 100 et 2000 ppmv de NO, le reste étant de l'azote (N₂), conditionné à une pression comprise entre 10 et 250 bar abs, typiquement à plus de 100 bar abs (avant début de soutirage).
- la source de NO contient un mélange gazeux NO/N₂ contenant entre 100 et 1000 ppmv de NO, le reste étant de l'azote (N₂), conditionné à une pression comprise entre 10 et 250 bar abs, typiquement à plus de 100 bar abs (avant début de soutirage).
- la source de NO est une (ou des) bouteille de gaz sous pression.
- la source de NO est une (ou des) bouteille de gaz ayant une contenance comprise entre 0,5 et 50 L (équivalent en eau).
- la bouteille de gaz comprend un corps cylindrique en acier ou en alliage d'aluminium.
- la bouteille de gaz est équipée d'un robinet simple (sans détendeur) ou à détendeur intégré ou RDI.
- la bouteille de gaz est équipée d'un RDI protégé par un capotage de protection, par exemple en métal ou polymère.
- le circuit patient comprend une branche inspiratoire et une branche expiratoire.
- le circuit patient comprend des conduites flexibles formant la branche inspiratoire et la branche expiratoire, typiquement des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire.
- la branche inspiratoire et la branche expiratoire comprennent des conduites flexibles, par exemple en polymère.
- la branche inspiratoire et la branche expiratoire sont en outre fluidiquement raccordées à, respectivement, des orifices de sortie et d'entrée du ventilateur médical.
- la branche inspiratoire du circuit patient peut comprendre un humidificateur de gaz.
- l'humidificateur de gaz est agencé en aval du module d'injection de NO de manière à pouvoir humidifier le gaz avant son administration par inhalation au patient.

Selon un autre aspect, l'invention concerne aussi une méthode de traitement thérapeutique d'une personne, i.e. un patient (adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne (i.e. être humain) en ayant besoin, d'un mélange gazeux comprenant de 1 à 80 ppmv de NO et au moins 21 %vol. d'oxygène au moyen d'une installation de fourniture de gaz, telle celle décrite ci-avant, comprenant un dispositif de délivrance de NO équipé du système de dosage de secours de NO selon l'invention, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

L'invention va maintenant être mieux comprise grâce à la description détaillée suivante, faite à titre illustratif mais non limitatif, en référence aux figures annexées parmi lesquelles :
- Fig. 1 schématise un mode de réalisation d'une installation de délivrance de gaz comprenant un dispositif de délivrance de NO équipé d'un système de dosage d'urgence de NO selon la présente invention ;
- Fig. 2 schématise l'association entre orifice calibré et actionneur d'un mode de réalisation du système de dosage d'urgence de NO de Fig. 1 ; et
- Fig. 3 à Fig. 5 schématisent le fonctionnement de l'association orifice calibré/actionneur de Fig. 2.

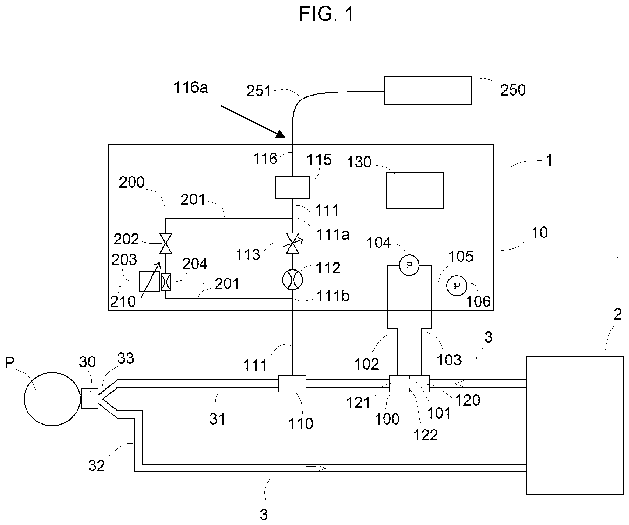

Fig. 1 schématise un mode de réalisation d'une installation de délivrance de gaz 1, 2 selon la présente invention comprenant un dispositif de délivrance de NO 1 comprenant un mécanisme de dosage d'urgence de NO, associé à un ventilateur mécanique 2, c'est-à-dire un appareil respiratoire délivrant un gaz respiratoire, laquelle installation est configurée pour délivrer du NO sous forme gazeuse à un patient à une concentration désirée correspondant à une posologie fixée par un médecin-anesthésiste ou analogue, typiquement entre 1 et 80 ppmv de NO (i.e. ppm en volume).

Le ventilateur médical 2 délivre un gaz respiratoire contenant au moins 21% d'oxygène, tel que de l'air ou un mélange NO/N₂, dans un circuit patient 3, en particulier dans une branche inspiratoire 31 dudit circuit patient 3, servant à acheminer et à fournir le gaz respiratoire à un patient P pendant ses phases inspiratoires, c'est-à-dire de fournir une assistance respiratoire au patient P, et à convoyer les gaz expirés par le patient lors de ses phases expiratoires.

Le ventilateur médical 2 est un appareil d'assistance respiratoire classique pouvant comprendre, selon le mode de réalisation désiré, soit une soufflante motorisée, aussi appelée turbine ou compresseur, soit une ou des vannes proportionnelles, en lieu et place de la soufflante motorisée ; dans ce cas, il est alimenté en gaz, par exemple en air médical, par une prise murale alimentée par un réseau hospitalier véhiculant le gaz au sein d'un établissement hospitalier.

Dans tous les cas, le ventilateur médical 2 délivre le gaz respiratoire dans le circuit patient 3. Son fonctionnement est par ailleurs contrôlé par une (ou des) carte électronique de commande ou analogue, et il est alimenté électriquement par des moyens d'alimentation électrique, telle secteur (110/220V) et/ou une batterie interne.

Par exemple, le ventilateur médical 2 peut être le Servo-n Néonatal^{®} de la société Getinge, qui est un ventilateur à vannes proportionnelles incluant une fonction HFO.

Comme visible sur Fig. 1, le circuit patient 3 comprend ici une branche inspiratoire 31 et une branche expiratoire 32 reliées fluidiquement à une pièce de jonction 33, telle une pièce en Y ou similaire, en communication fluidique avec une interface respiratoire 30 permettant de délivrer le gaz au patient P ou bien de collecter les gaz expirés par ledit patient P. L'interface respiratoire 30 peut par exemple être un masque facial, une sonde d'intubation ou autre.

Les branches inspiratoire 31 et expiratoire 32 comprennent des conduits, canalisations, tuyaux, passages, tubulures ou similaires, par exemple des tuyaux flexibles en polymère, aptes à et configurés pour convoyer les flux gazeux.

Le gaz respiratoire circulant dans la branche inspiratoire 31 du circuit patient 3, c'est-à-dire allant du ventilateur mécanique 2 au patient P, est inhalé par le patient P tandis que les gaz expirés par ledit patient P, i.e. des gaz enrichis en CO₂, empruntent la branche expiratoire 32 du circuit patient 3 en direction du ventilateur mécanique 2 pour être déchargés à l'atmosphère par le ventilateur mécanique 2.

Par ailleurs, un capteur de débit 100 ainsi qu'un module d'injection de NO 110 sont disposés dans la branche inspiratoire 31 du circuit patient 3. Le capteur de débit 100 est préférablement disposé dans la branche inspiratoire 31 entre le module d'injection de NO 110 et le ventilateur mécanique 2, de manière à pouvoir y mesurer le débit de gaz qui y circule, typiquement un flux d'air provenant du ventilateur mécanique 2.

La branche inspiratoire 31 peut également comprendre un humidificateur (non représenté) afin d'humidifier le gaz délivré au patient P. Préférablement, l'humidificateur est placé en aval du module d'injection de NO 110, c'est-à-dire entre ledit module d'injection de NO 110 et l'interface respiratoire 30 fournissant le gaz au patient P.

Plus précisément, le capteur de débit 100 est utilisé afin de mesurer le flux gazeux, i.e. un débit, délivré par le ventilateur mécanique 2 et circulant dans la branche inspiratoire 31, par exemple un capteur de débit massique ou à mesure de pression différentielle. Ces mesures de débit servent à contrôler la quantité de NO apportée par le dispositif de délivrance de NO 1, comme expliqué ci-après.

Dans le mode de réalisation de Fig. 1, le capteur de débit 100 est du type à mesure de pression différentielle, c'est-à-dire que le capteur de débit 100 comprend une restriction interne 101 qui crée une perte de charge engendrant un différentiel ou gradient de pression lorsqu'un débit gazeux traverse cette restriction interne 101.

Plus précisément, comme on le voit en Fig. 1, le capteur de débit 100 comprend des chambres amont 120 et aval 121 qui sont séparées par une paroi 122 traversées un passage de gaz de sorte de former la restriction interne 101. Une ligne amont 103 et une ligne aval 102 de mesure de pression sont connectées fluidiquement au capteur de débit 100 en des sites de raccordement situés en amont et en aval de la restriction interne 101, en particulier aux chambres amont 120 et aval 121, afin d'y opérer les mesures de pression du flux circulant, avant et après perte de charge, typiquement de l'air ou un mélange O₂/N₂.

La différence de pression créée par la restriction interne 101 est déterminée par un capteur de pression différentiel 104 raccordé au capteur de débit 100 par le biais des lignes amont 102 et aval 103 qui forment des conduits de mesure de pression et fournissent au capteur de pression différentiel 104, les mesures de pression du flux circulant, avant et après perte de charge. Préférentiellement, le capteur de pression différentiel 104 est intégré dans le boitier 10 du dispositif de délivrance de NO 1, comme illustré en Fig. 1. Le capteur 104 peut être par ailleurs soit connecté électriquement à une unité de pilotage 130, aussi appelée moyens de pilotage 130, soit peut lui transmettre les mesures de pression afin qu'elles y soient traitées informatiquement.

Par ailleurs, une ligne de dérivation 105 vient se connecter à la ligne de pression aval 103 afin de convoyer l'information de pression régnant dans ladite ligne de pression aval 103 à un capteur de pression 106, typiquement de type relatif. Ce capteur de pression 106 mesure la pression régnant dans la chambre amont 120 du capteur de débit 100 et peut retourner cette valeur, via une connexion électrique, à l'unité de pilotage à des fins de compensation, considérant que la valeur de débit réelle traversant le capteur de débit 100 dépend principalement de la mesure de pression différentielle 104 mais est également affectée par la pression relative 106 régnant en amont dudit capteur de débit 100.

L'unité de pilotage 130, i.e. les moyens de pilotage 130, comprend un système de traitement de données, i.e. de mesures, comprenant typiquement un (ou des) microprocesseur(s) agencé sur une (ou des) carte électronique et mettant en oeuvre un (ou des) algorithme(s), i.e. un (ou des) programme d'ordinateur. En d'autres termes, l'unité de pilotage 130 est configurée pour traiter et/ou exploiter les mesures, c'est-à-dire les signaux de mesure de pression ou les valeurs de pression, transmises par le capteur de pression différentielle 104 (et capteur de pression 106) coopérant avec le capteur de débit 100.

Bien entendu, l'unité de pilotage 130 peut être aussi configurée pour piloter d'autres éléments électromécaniques intégrés dans le boitier 10 ou carcasse externe du dispositif de délivrance de NO 1.

En particulier, l'unité de pilotage 130 dispose d'une table de correspondance préenregistrée, i.e. mémorisée, qui permet la détermination du débit de gaz circulant dans la branche inspiratoire 31 et au travers du capteur de débit 100, c'est-à-dire de transformer une valeur de pression transmise par le capteur de pression différentiel 104 en valeur de débit traversant le capteur de débit 100 (éventuellement compensée de la valeur retournée par le capteur de pression 106). Cette détermination du débit du flux gazeux (e.g. air) traversant le capteur de débit 100 permet ensuite de calculer la quantité de NO devant être ajoutée au gaz circulant dans la branche inspiratoire 31 avant d'atteindre le patient P et ce, afin de pouvoir délivrer le NO sous forme gazeuse au patient P à la concentration désirée correspondant à la posologie fixée par un médecin-anesthésiste ou analogue, typiquement entre 1 et 80 ppmv de NO (i.e. ppm en volume).

En d'autres termes, en utilisant la mesure de pression retournée par le capteur de pression différentielle 104 et la table de correspondance, l'unité de pilotage 130 peut déterminer le débit de gaz (e.g. air ou N₂/O₂ avec teneur en O₂ > 21 % vol.) issu du ventilateur mécanique 2 et la quantité de NO devant être ajoutée, via le module d'injection de NO 110, afin d'obtenir la concentration de NO souhaitée, i.e. la posologie définie par le médecin, devant être inhalée par le patient P.

Le mélange gazeux final obtenu au niveau du module d'injection de NO 110 comprend alors principalement de l'azote (N₂), de l'oxygène (O₂) en une teneur d'au moins 21 %vol., et du NO à une teneur typiquement comprise entre 1 et 80 ppmv.

Plus précisément, en fonction du débit gazeux (i.e. air ou N₂/O₂) circulant dans la branche inspiratoire 31 ayant été déterminé à l'aide du capteur de débit 100, l'unité de pilotage 130 détermine la quantité de NO, typiquement un mélange de NO et de N₂, devant être ajouté au gaz (e.g. air ou N₂/O₂ avec teneur en O₂ > 21% vol.) circulant dans la branche inspiratoire 31 par le module d'injection de NO 110, afin d'obtenir la concentration en NO finale souhaitée.

Le dispositif de délivrance de NO 1 est alimenté en NO gazeux, typiquement en mélange NO/N₂ gazeux, provenant d'une source de NO 250 reliée fluidiquement au dispositif de délivrance de NO 1, en particulier à une ligne haute pression 116 dudit dispositif de délivrance de NO 1, par une ligne d'alimentation 251, telle un conduit flexible ou analogue. Typiquement, la source de NO 250 est une (ou des) bouteille de gaz sous pression renfermant un mélange de NO/N₂ contenant une concentration en NO généralement comprise entre 100 et 2000 ppmv, de préférence entre 200 et 1000 ppmv, par exemple de l'ordre de 800 ppmv, et conditionné à une pression (lorsque totalement pleine) pouvant atteindre 200 à 250 bar abs, voire plus.

Le mélange NO/N₂ est fourni au module d'injection 110 par le dispositif de délivrance de NO 1 via une ligne d'injection 111, telle une conduite de gaz flexible.

La ligne d'injection 111 est fluidiquement connectée à une ligne haute pression 116 du dispositif de délivrance de NO 1, laquelle ligne haute pression 116 présente une entrée haute pression 116a connectée fluidiquement à la source de NO pour être alimentée en NO/N₂ sous pression, c'est-à-dire à une pression pouvant atteindre 200 bar abs.

La ligne haute pression 116, par exemple un passage ou conduit de gaz, est agencée dans le boitier 10 du dispositif de délivrance de NO 1 et comprend un régulateur de pression 115 qui réduit la pression du mélange NO/N₂ à une valeur stable, par exemple environ 4 bar abs ou toute autre pression adéquate. Le port de sortie du régulateur de pression 115 fourni donc une pression stable dans la portion amont de la ligne d'injection 111.

Un dispositif à vanne 113, préférentiellement une électrovanne, avantageusement une électrovanne proportionnelle, telle que la série VSO miniature de Parker par exemple, est agencé dans le dispositif 1 afin de contrôler le débit de NO gazeux au sein de la ligne d'injection 111. Le débit gazeux circulant dans la ligne d'injection 111 est mesuré par un dispositif de mesure de débit 112, aussi appelé capteur de débit de NO, agencé sur la ligne d'injection 111, préférentiellement placé en aval de l'électrovanne 113, comme visible sur Fig. 1.

Le régulateur de pression 115, le dispositif à vanne ou électrovanne 113, le capteur de débit de NO 112 et une portion de la ligne d'injection 111 sont donc agencés dans le boitier 10 du dispositif de délivrance de NO 1.

Le dispositif à vanne 113 est configuré pour être normalement dans une position fermée (i.e. un état fermé) pour empêcher toute circulation de gaz dans la ligne d'injection 111. Pour passer en position ouverte, le dispositif à vanne 113 doit être commandé par les moyens de pilotage 130, comme c'est le cas pendant un fonctionnement normal du dispositif de délivrance de NO 1.

Par ailleurs, on prévoit un système de dosage de secours 200 de NO agencé dans le boitier 10 du dispositif de délivrance de NO 1, lequel est configuré pour fonctionner en cas de dysfonctionnement du dispositif de délivrance de NO 1, comme expliqué ci-après.

Le système de dosage de secours 200 de NO comprend une ligne de secours 201, aussi appelée ligne de bipasse, tel un passage de gaz, un conduit de gaz ou analogue. La ligne de secours 201 vient se connecter fluidiquement à la ligne d'injection 111 en un premier site de raccordement 111a situé en amont du dispositif à vanne 113, telle une électrovanne proportionnelle, et ici en aval du régulateur de pression 115, et en un second site de raccordement 111b situé en aval du dispositif à vanne 113 et, de préférence, en aval du capteur de débit de NO 112.

Autrement dit, un dispositif à vanne 113, telle une électrovanne proportionnelle, et préférentiellement le capteur de débit de NO 112 sont situés entre les premier et second sites de raccordement 111a, 111b de la ligne de secours 201, c'est-à-dire que la ligne de secours 201 bipasse, le dispositif à vanne 113 et préférentiellement le capteur de débit de NO 112 situés sur la ligne d'injection 111. Selon un autre mode de réalisation, le second site de raccordement 111b peut être situé en aval du dispositif à vanne 113 et en amont du capteur de débit de NO 112, c'est-à-dire entre ces deux éléments.

La ligne de secours 201 comprend quant à elle une électrovanne de secours 202 et un dispositif de contrôle de débit 210 faisant partie du système de dosage de secours 200 de l'invention. Cette électrovanne de secours 202 est configurée pour être normalement dans une position ouverte (i.e. état ouvert) pour permettre une circulation de gaz dans la ligne de secours 201. Pendant un fonctionnement normal du dispositif de délivrance de NO 1, cette électrovanne de secours 202 est donc commandée par les moyens de pilotage 130 pour être en position fermée (i.e. état fermé) pour empêcher que le flux de NO/N2 n'emprunte la ligne de secours 201.

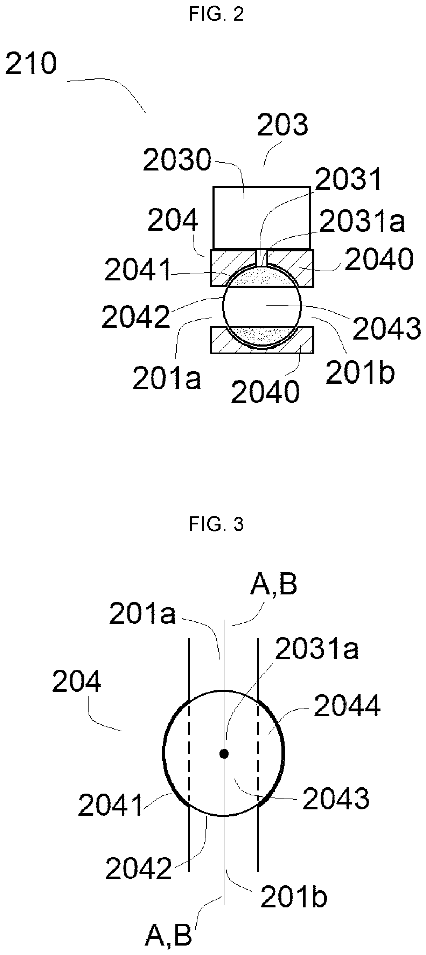

Le dispositif de contrôle de débit 210 forme ou comprend un système à orifice calibré 204 de type proportionnel. Il peut revêtir différentes formes, i.e. agencements, notamment celui illustré sur Fig. 2 à Fig. 5 et détaillé ci-après.

L'électrovanne de secours 202 est préférentiellement une électrovanne de type « tout ou rien » ayant deux états possibles, à savoir un état ouvert et un état fermé, par exemple une électrovanne de la série Picosol de IMI Norgren, ou bien de la série HDI de The Lee Company. Comme déjà dit, l'électrovanne de secours 202 est normalement ouverte, c'est-à-dire qu'en l'absence d'une commande électrique venant de l'unité de pilotage 130, l'électrovanne de secours 202 est en état ouvert, i.e. position ouverte, ce qui permet alors au gaz issu de la source de NO d'emprunter la ligne de secours 201 depuis le premier site de raccordement 111a en direction du second site de raccordement 111b. Ce sont les moyens de pilotage ou unité de pilotage 130, qui assurent la fermeture de l'électrovanne de secours 202, c'est-à-dire son passage de l'état ouvert à l'état fermé, i.e. en position fermée, pour empêcher toute circulation de gaz dans la ligne de secours 201 lorsqu'un tel flux n'est pas souhaité, c'est-à-dire en fonctionnement normal.

Autrement dit, les moyens de pilotage 130, i.e. l'unité de pilotage, sont configurés pour coopérer avec l'électrovanne de secours 202, le dispositif de contrôle de débit 210, le dispositif à vanne 113 et le dispositif de mesure de débit 112, lors d'un fonctionnement normal du dispositif de délivrance de NO 1.

Dans le mode de réalisation présenté sur Fig. 2 à Fig. 5, le dispositif de contrôle de débit 210 formant ou comprenant un système à orifice calibré proportionnel, c'est-à-dire constituant, comprenant ou formant un orifice calibré 204 de type proportionnel, comprend un moyen actionneur 203 coopérant avec un élément mobile 2042 agencé dans un compartiment 2041, ledit élément mobile 2042 comprenant un évidement traversant 2043.

Toutefois, selon d'autres modes de réalisation, le dispositif de contrôle de débit 210 constituant l'orifice calibré 204 de type proportionnel peut revêtir une autre forme, par exemple un dispositif de type à vanne à pointeau ou encore un ensemble comprenant un régulateur de pression pouvant être associé à d'autres composants et être réglé à différentes pressions de sortie, pour générer ainsi différents débits.

Ainsi, Fig. 2 est un schéma en coupe d'un mode de réalisation du dispositif de contrôle de débit 210, i.e. de l'orifice calibré 204, qui est formé ici par le moyen actionneur 203 et l'élément mobile 2041 associé à l'évidement traversant 2043, du système de dosage d'urgence de NO équipant le dispositif de délivrance 1 de NO de l'invention.

Le moyen actionneur 203, plus simplement appelé actionneur, de Fig. 2 est préférentiellement un moteur de type pas à pas 2030, par exemple comme celui commercialisé par la société Portescap, prolongé par un axe 2031 mécaniquement couplé en 2031a à l'élément mobile 2042.

Par ailleurs, l'élément mobile 2042 est ici une sphère. La sphère formant l'élément mobile 2042 peut être métallique, par exemple en acier inoxydable et présente un évidement traversant 2043, c'est-à-dire qu'elle est diamétralement traversée par un perçage ou passage interne permettant le passage du gaz. L'élément mobile 2042, i.e. la sphère, est logé dans un compartiment ou logement interne 2041 formant une chambre à sphère qui est ici de forme générale sphérique. Le logement 2041 est aménagé dans une pièce formant corps 2040. Le diamètre externe de la sphère 2042 est sensiblement égal au diamètre interne du logement interne 2041.

La pièce formant corps 2040 peut être aussi métallique, par exemple une bille d'acier ou analogue. Elle comprend un port d'entrée 201a et un port de sortie 201b en communication fluidique avec le logement interne 2041.

Sur Fig. 2, on voit que l'évidement traversant 2043 de la sphère 2042 est aligné avec les ports d'entrée 201a et de sortie 201b du corps 2040, qui sont en communication fluidique avec la ligne de secours 201, c'est-à-dire qu'ils sont en continuité fluidique, de sorte que le gaz peut s'écouler du port d'entrée 201a vers le port de sortie 201b via l'évidement traversant 2043 de la sphère 2042.

Comme indiqué, le moyen actionneur 203 est ici un moteur pas à pas 2030 entraînant en rotation l'axe 2031 et donc la sphère 2042. En réponse à une commande provenant de l'unité de pilotage 130, le moteur 2030 pas à pas va adopter une position différente et faire subir une rotation à l'axe 2031 qui va alors entraîner la sphère 2042 aussi en rotation.

En considérant que l'unité de pilotage 130 est en capacité de faire varier la valeur de commande de façon proportionnelle, il s'ensuit que l'axe 2031 peut subir des mouvements de rotation plus ou moins importants et de façon proportionnelle, par exemple entre 0 et 90°. Autrement dit, le mouvement de rotation subi par l'axe 2031 est donc transmis à la sphère 2042 qui pivote en réponse, au sein de son logement 2041, comme illustré sur Fig. 3 à Fig. 5, ce qui permet de régler ou ajuster le débit de gaz souhaité, pendant le fonctionnement normal de l'appareil 1.

Ainsi, Fig. 3 est un schéma de dessus de l'orifice calibré 204 de Fig. 2 montrant, comme déjà expliqué, l'axe 2031 qui impose à la sphère 2042 de présenter son évidement 2043 en continuité des ports d'entrée 201a et de sortie 201b du corps 2040 de sorte à créer une connexion fluidique entre lesdits ports d'entrée 201a, 201b et autoriser ainsi le passage de gaz au travers des ports 201a, 201b et de l'évidement 2043. L'orifice calibré 204 est alors à son ouverture maximale, c'est-à-dire en position totalement ouverte. Dans cette position, l'axe AA de l'évidement traversant 2043 de la sphère 2042 est (quasi)coaxial à l'axe BB passant par les ports d'entrée (201a) et de sortie (201b) de sorte que l'ouverture soit maximale, donc le flux maximal dans la ligne de secours 201, y compris au travers de l'évidement traversant 2043 de la sphère 2042.

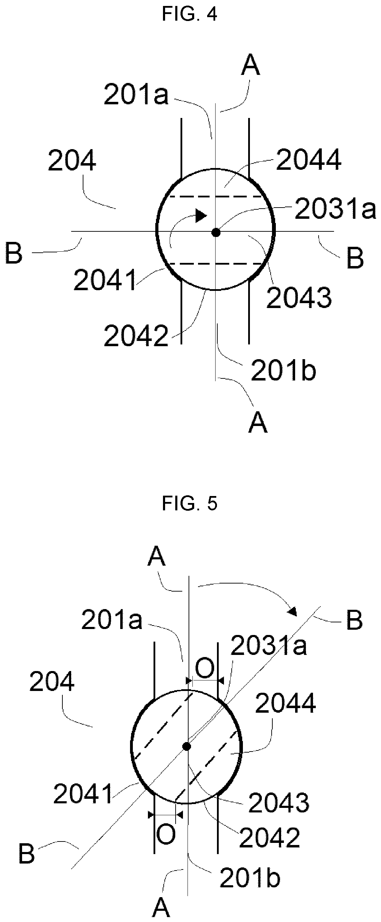

En Fig. 4, l'unité de pilotage 130 a commandé le moteur pas à pas 2030 pour faire subir à l'axe 2031, une rotation ici de l'ordre de 90° et par conséquent aussi à la sphère 2042 qui subit aussi la même rotation de 90°. Après rotation, les ports d'entrée 201a et de sortie 201b du corps 2040 de l'orifice calibré 204 ne font plus face à l'évidement 2043 de la sphère 2042 mais à une portion 2044 non-évidée, i.e. pleine, de la sphère 2042 et se retrouvent alors totalement obturés par la partie non-évidée 2044 de la sphère 2042. La connexion fluidique est alors rompue et aucun gaz ne peut circuler entre les ports d'entrée 201a et de sortie 201b du corps 2040 de l'orifice calibré 204. L'orifice calibré 204 est alors totalement fermé.

Dans cette position dite fermée, l'axe AA de l'évidement traversant 2043 de la sphère 2042 est (quasi)perpendiculaire à l'axe BB passant par les ports d'entrée (201a) et de sortie (201b) de sorte qu'aucun passage de gaz n'a lieu au travers de l'évidement traversant 2043, donc dans la ligne de secours 201.

Entre Fig. 3 et Fig. 4, l'unité de pilotage 130 a imposé à l'actionneur 203 des valeurs de commandes extrêmes, i.e. entre 0° et 90° de rotation, permettant d'obtenir soit une communication complète (cf. Fig. 3), soit une isolation complète (cf. Fig. 4) des ports d'entrée 201a et de sortie 201b du corps 2040 de l'orifice calibré 204.

Toutefois, l'unité de pilotage 130 est aussi configurée pour pouvoir assigner, de façon proportionnelle, des commandes entraînant une rotation de l'axe 2031 et de la sphère 2042 entre ces deux positions angulaires extrêmes, i.e. 0° et 90° de rotation, c'est-à-dire un angle non nul mais inférieur à 90°.

Ainsi, Fig. 5 donne l'exemple une position angulaire intermédiaire où la sphère 2042 a subi un mouvement de rotation de l'ordre de 45°. Dans ce cas, le port d'entrée 201a du corps 2040 de l'orifice calibré 204 est partiellement obstrué, c'est-à-dire exposé à des parties non-évidée 2044 et évidée 2043 de la sphère 2042. La section de passage de gaz de la partie évidée 2043 de la sphère 2042 en relation fluidique avec le port d'entrée 201a est alors définie par un niveau (ou taille) d'ouverture O. Cette section de passage de gaz est toujours inférieure à la section maximale de connexion fluidique telle que représentée en Fig. 3. Par le biais de la rotation axiale, le même niveau d'ouverture O apparaît entre le port de sortie 201b et la partie évidée 2043 de la sphère 2042 du corps 2040 de l'orifice calibré 204.

Dans les positions dites intermédiaires, l'axe AA de l'évidement traversant 2043 de la sphère 2042 et l'axe BB passant par les ports d'entrée (201a) et de sortie (201b) forment entre eux un angle variable compris strictement ici entre 0 et 90° de sorte que le passage de gaz au travers de l'évidement traversant 2043, donc dans la ligne de secours 201, soit limité/réduit mais non-nul, ni maximum, c'est-à-dire en fonction de l'ouverture O désirée de l'orifice calibré 204.

Ainsi, en fonction de la commande imposée sur l'actionneur 203 par l'unité de pilotage 130, le niveau d'ouverture O défini par l'intersection des ports d'entrée 201a, de sortie 201b et de la partie évidée 2043 de la sphère 2042, varie d'une valeur nulle (Fig. 3) à une valeur maximale (Fig. 4), c'est-à-dire peut prendre les valeurs intermédiaires situées entre ces deux valeurs extrêmes, i.e. entre 0 et 90°, ce qui permet de régler ou ajuster le débit de gaz circulant dans la ligne de secours 201.

En effet, on comprend aisément que chaque niveau ou valeur d'ouverture O correspond à un orifice calibré équivalent dont le diamètre de passage du gaz est fonction du positionnement de la sphère 2042 et par conséquent de la commande envoyée par l'unité de pilotage 130, i.e. les moyens de pilotage, à l'actionneur 203.

Comme déjà dit, cet ensemble forme donc bien un système à orifice calibré proportionnel puisque son calibre ou niveau d'ouverture O varie en fonction de la position angulaire prise par la sphère 2042 au sein du corps 2040 de l'orifice calibré 204.

Or, la pression régnant dans la portion amont de la ligne de secours 201, c'est-à-dire en amont de l'orifice calibré 204, est stable et connue puisqu'elle correspond à la pression de détente du régulateur de pression 115 fixée par exemple à environ 4 bar abs. Le débit de gaz circulant dans la portion aval de la ligne de secours 201, c'est-à-dire en aval de l'orifice calibré 204, est donc fonction du niveau d'ouverture O.

Ainsi, l'unité de pilotage 130 peut disposer d'une table de correspondance reliant un niveau de commande donné à un niveau d'ouverture et à un débit de gaz traversant l'orifice calibré 201 en direction de la ligne d'injection 111 et y pénétrant au second site de raccordement 111b.

Pour des raisons de simplification, on admet que le niveau de pression régnant dans la branche inspiratoire 31 du circuit patient 3, et donc dans le module d'injection de NO 110 et la ligne d'injection 111 est négligeable au regard de la pression de détente du régulateur de pression 115 et n'a donc pas d'impact sur la précision des mesures de débit circulant dans la ligne de secours 201 réalisées par l'unité de pilotage 130.

Bien entendu, selon un mode de réalisation particulier, des moyens de mesure supplémentaires, tel qu'un dispositif de mesure de pression additionnel agencé pour opérer une mesure de pression en aval de l'orifice calibré 204 de la ligne de secours 201, peuvent être implémentés, i.e. utilisés, à des fins de compensation, sans changer l'objet de la présente invention.

Enfin, il est à noter que le choix d'un moteur pas à pas est particulièrement recommandé car, contrairement aux électrovannes 202, 113 qui prendront une position de repos, en cas de coupure d'alimentation, à savoir une position ouverte pour l'électrovanne tout ou rien 202 et une position fermée pour l'électrovanne proportionnelle 113, la position du moteur pas à pas reste permanente et fixée selon la dernière commande imposée. Autrement dit, l'orifice calibré 204 présente un niveau d'ouverture fixe correspondant à la dernière valeur de commande reçue par le moteur pas à pas, i.e. la dernière commande provenant des moyens de pilotage 130.

Bien entendu, la présente invention ne se limite pas à un actionneur de type moteur pas à pas. En effet, tout autre actionneur gardant sa position en cas de défaut d'alimentation électrique et pouvant être couplé à un mécanisme mécanique permettant de définir ou constituer un système à orifice calibré à taille variable peut être utilisé, comme par exemple un moteur linéaire ou autre.

Par ailleurs, le dispositif de délivrance de NO 1 est alimenté électriquement par une alimentation électrique, telle secteur (110/220V) ou une batterie interne, afin de permettre le bon fonctionnement de ses composants nécessitant du courant électrique pour fonctionner, notamment l'actionneur 203, tel un moteur électrique pas à pas, l'unité de pilotage 130, les électrovannes 202, 113, ou autres.

En outre, le dispositif de délivrance de NO 1 comprend aussi des moyens de mémorisation, telle une mémoire informatique, pour mémoriser des données, informations ou autres, par exemple une ou des tables de correspondances, les mesures de débit de gaz opérées par le dispositif de mesure de débit 112, ou autres.

En cas de défaillance majeure du fonctionnement du dispositif de délivrance de NO 1, causée par exemple par une rupture de son câble d'alimentation électrique occasionnée par des vibrations dans le cadre d'un transport de patient par exemple ou en cas de défaut d'alimentation électrique, on doit pouvoir continuer à assurer un traitement du patient avec du NO inhalé et ce, malgré le dysfonctionnement engendrant un arrêt de fonctionnement des moyens de pilotage 130, du fait par typiquement d'un défaut d'alimentation électrique.

A cette fin, le dispositif 1 de l'invention est configuré pour qu'en cas d'une telle défaillance, l'électrovanne de secours 202 passe en position ouverte pour permettre une circulation de gaz dans la ligne de secours 201, alors que, dans le même temps, le dispositif à vanne 113 passe en position fermée pour stopper toute circulation de gaz dans la ligne d'injection 111, ce qui permet de fournir, via la ligne de secours 201 et le dispositif de contrôle de débit 210, le gaz à un débit gazeux de secours préfixé.

En fait, pendant le fonctionnement normal du dispositif 1 précédant le dysfonctionnement, selon l'invention, ledit débit gazeux de secours est déterminé par les moyens de pilotage 130 à partir d'une ou des mesures de débit du gaz fournies par le dispositif de mesure de débit 112, aux moyens de pilotage 130. Les moyens de pilotage 130 peuvent alors prérégler le dispositif de contrôle de débit 210 de sorte qu'il puisse délivrer le gaz au débit gazeux de secours préfixé.

Autrement dit, les moyens de pilotage 130 déterminent le débit gazeux de secours devant être administré en cas de panne ou autre dysfonctionnement, à partir des mesures de débit du gaz fournies par le dispositif de mesure de débit 112 lors du fonctionnement normal du dispositif 1, et agissent sur le dispositif de contrôle de débit 210 pour y régler ce débit gazeux de secours préfixé, par exemple en jouant sur le calibre ou niveau d'ouverture O agissant sur la position angulaire prise par la sphère 2042 au sein du corps 2040 de l'orifice calibré 204, comme expliqué ci-avant.

Plus précisément, le fonctionnement du système de dosage de secours 200 de NO du dispositif de délivrance de NO 1 de l'invention est globalement le suivant.

Comme illustré en Fig. 1, le dispositif de délivrance de NO 1 coopère avec un ventilateur mécanique 2 afin d'apporter une aide thérapeutique au patient P. Comme déjà expliqué, le débit de gaz (i.e. air ou N₂/O₂) issu du ventilateur mécanique 2 et circulant dans la branche inspiratoire 31 du circuit patient 3 est mesuré en permanence par le capteur de débit 100 et l'unité de pilotage 130. Cette mesure de débit permet à l'unité de pilotage 130 de déterminer, en temps réel, le débit de NO devant être injecté dans la ligne d'injection 111 et le module d'injection de NO 110 afin de satisfaire la concentration de NO souhaitée dans le gaz fourni au patient, à savoir typiquement entre 5 et 80 ppmv.

En fonctionnement normal, afin de ne pas introduire de débit additionnel provenant de la ligne de secours 201 dans la ligne d'injection 111, l'unité de pilotage 130 commande l'électrovanne 202, qui est préférentiellement de type tout ou rien, en position fermée et, en parallèle, va piloter l'actionneur 203, i.e. moteur pas à pas, afin de prérégler l'orifice calibré 204 en définissant un niveau d'ouverture O donné.

Ceci est opéré de la façon suivante par l'unité de pilotage 130, à partir d'une ou plusieurs mesures de débit provenant du dispositif de mesure de débit 112, comme déjà expliqué.

L'unité de pilotage 130 réalise d'abord une moyenne du débit de NO (i.e. du mélange NO/N₂) ayant circulé dans la ligne d'injection 111 pendant un temps donné, par exemple pendant 1 minute (ou sur une période de temps plus longue mais le débit doit alors être converti en L/min), pendant un fonctionnement normal du dispositif 1. L'unité de pilotage 130 évalue donc une valeur de débit de NO moyen fixe (en L/min) permettant de se rapprocher de la concentration en NO souhaitée.

Lorsque cette valeur a été déterminée, l'unité de pilotage 130 réalise une conversion par le biais de sa table de correspondance mémorisée de manière à commander l'actionneur 203 du dispositif de contrôle de débit 210 et par conséquent définir un niveau d'ouverture de l'orifice calibré 204 afin d'autoriser un débit de NO circulant dans la ligne de secours égal à la valeur calculée de NO moyen fixe. C'est cette valeur calculée de NO moyen qui va servir de débit gazeux de secours en cas de dysfonctionnement de l'appareil 1.

Il est à noter que cette activité n'a pas d'effet physique, i.e. aucun débit de gaz ne circule dans la ligne de secours 201 car l'électrovanne tout ou rien 202 est fermée.

Dès lors, en cas de défaillance majeure du dispositif de délivrance de NO 1 et/ou d'interruption de son alimentation électrique, à l'exception de l'actionneur 203 et du régulateur de pression 115 qui a un fonctionnement purement pneumatique, l'ensemble des actionneurs électromécaniques, en particulier les électrovannes, retournent à leur position de repos et l'unité de pilotage 130, ainsi que les différents capteurs se retrouvent sans alimentation électrique, donc sans capacité à communiquer et/ou à piloter/commander d'autres composants.

Comme déjà indiqué, l'électrovanne proportionnelle 113 retrouve sa position de repos, à savoir sa position fermée empêchant tout passage de gaz, alors que l'électrovanne 202 se retrouve simultanément dans sa position de repos, à savoir sa position ouverte, autorisant le passage du gaz provenant de la source de NO dans la ligne de secours 201 et sa circulation jusqu'à atteindre le second site de jonction 111b, puis la partie aval de la ligne d'injection 111.

Le mélange NO/N₂ circule donc dans la ligne de secours 201 au débit de secours préfixé qui est contrôlé par l'orifice calibré, à savoir la dernière valeur valide du débit de NO moyen fixe ayant été déterminée par l'unité de pilotage 130 lors du fonctionnement normal du dispositif 1, préalablement à son dysfonctionnement.

Le débit de secours de NO/N₂ qui rejoint la ligne d'injection 111 (en 111b) peut ensuite être injecté dans la branche inspiratoire 31 du circuit patient 3 via le module d'injection de NO 110, comme déjà expliqué.

Autrement dit, selon l'invention, le dispositif de contrôle de débit 210 est configuré pour fournir le gaz, i.e. NO/N₂, à un débit gazeux de secours préfixé, où ledit débit gazeux de secours est déterminé par les moyens de pilotage 130 à partir d'une (ou plusieurs) mesure de débit du gaz fournie par le dispositif de mesure de débit 112, pendant un fonctionnement normal du dispositif 1 précédant le dysfonctionnement, par exemple la dernière valeur de débit ayant été mesurée avant le dysfonctionnement affectant le bon fonctionnement du dispositif 1.

Cette valeur de débit est préréglée au sein du dispositif de contrôle de débit 210, par exemple en agissant sur la position angulaire prise par la sphère 2042 au sein du corps 2040 de l'orifice calibré 204 du dispositif de contrôle de débit 210 afin d'y faire varier le calibre ou niveau d'ouverture O, comme expliqué ci-avant, par commande dudit dispositif de contrôle de débit 210 par les moyens de pilotage 130, ledit préréglage ayant lieu, c'est-à-dire étant opéré ou réalisé, pendant ledit fonctionnement normal du dispositif 1.

Bien entendu, le fait d'injecter un débit continu de NO dans la branche inspiratoire 31 du circuit patient 3 ne garantit pas une même précision de concentration en NO inhalé que lorsque le système de délivrance de NO 1 opère en fonctionnement normal, c'est-à-dire en ajustant le débit de NO en fonction du débit traversant le capteur de débit 100, mais le volume tampon généré par la portion de la branche inspiratoire 31 située en aval du module d'injection de NO, qui est éventuellement augmentée du volume de la chambre d'humidification lorsqu'elle est présente, permet de lisser les variations de concentration de NO inhalé par le patient et de se rapprocher de la valeur cible souhaitée, c'est-à-dire la posologie en NO.

Dans tous les cas, pouvoir s'approcher de la valeur cible de NO souhaitée grâce au système de dosage de secours 200 de NO intégré au dispositif de délivrance 1 de NO de l'invention améliore considérablement la sécurité pour le patient en comparaison avec un débit de NO de secours fixe (par exemple de 250 mL/min) usuellement délivré par le système de sécurité des dispositifs de délivrance de NO de l'art antérieur.

Ainsi, à titre comparatif, alors que le système de dosage de secours 200 de NO intégré au dispositif de délivrance 1 de NO de l'invention permet de garantir une concentration en NO sensiblement égale à la posologie souhaité, avec un système de secours basé sur un débit fixe de 250 mL/min, tel que classiquement mis en oeuvre dans les dispositifs de délivrance de NO de l'art antérieur :
- pour un débit moyen de NO nécessaire de 0.05 L/min pour assurer normalement une concentration en NO de 10 ppmv (cas d'utilisation en néonatologie avec ventilateur de type HFO), la concentration résultante avec le débit fixe de 250 mL/min, est de 50 ppmv, ce qui correspond à une multiplication par 5 de la posologie souhaitée.
- à l'inverse, pour un débit moyen nécessaire de NO de 1 L/min pour assurer 80 ppmv de concentration en NO (cas d'utilisation chez l'adulte, par exemple en cas d'hypertension pulmonaire pendant une chirurgie cardiaque), la concentration résultante chute à 20 ppmv, ce qui correspond à une diminution de 75% de la posologie souhaitée.

Dans les deux cas, les écarts importants de posologie peuvent induire des situations inacceptables et dangereuses pour le patient et ce, contrairement au système de dosage de secours 200 de NO intégré au dispositif de délivrance 1 de NO de l'invention qui permet lui de respecter la posologie désirée.

Autrement dit, le système de dosage de secours 200 de NO de l'invention présente des avantages indéniables en renforçant la sécurité des patients en :
- injectant de façon automatique un débit de secours de NO sans attendre que l'utilisateur ne se rende compte de la situation et intervienne en basculant sur le dosage pneumatique de secours.
- garantissant que la concentration de NO inhalé par le patient est similaire à la concentration souhaitée par le médecin, c'est-à-dire la posologie désirée.

Bien entendu, le basculement sur le système de dosage de secours 200 de NO de l'invention n'est que temporaire, c'est-à-dire ne dure que le temps nécessaire au remplacement de l'équipement ou composant défaillant qui a déclenché le système d'alarme sonore et/ou visuel afin d'alerter le personnel soignant.

Afin d'éviter l'activation à mauvais escient du système de dosage de secours 200 de NO, l'unité de pilotage 130 est en outre configurée pour procéder à des séquences d'initialisation et d'extinction adéquates. Par exemple, en cas d'arrêt voulu par l'utilisateur de la thérapie de NO, l'unité de pilotage 130 peut commander l'actionneur 203 afin de fermer l'orifice calibré 204. Ainsi, en cas d'extinction volontaire et donc d'ouverture de l'électrovanne 202, la configuration « fermée » de l'orifice calibré 204 interdit alors toute circulation de débit de NO dans la ligne de secours 201, le temps que le dispositif de délivrance de NO 1 soit à l'arrêt.

Le dispositif de délivrance de NO 1 équipé du système de dosage de secours 200 de NO de l'invention est particulièrement bien adaptée à la fourniture de mélange gazeux comprenant de 1 à 80 ppmv de NO et au moins 21%vol. d'oxygène à des patients (adultes, enfants, adolescents ou nouveau-nés), souffrant d'hypertensions pulmonaires et/ou d'hypoxie, qui peuvent engendrer des vasoconstrictions pulmonaires ou analogues, par exemple causés par des pathologies ou troubles pulmonaires de type PPHN (hypertension pulmonaire persistante du nouveau-né) ou SDRA (syndrome de détresse respiratoire aigüe), ou encore engendrés par une opération de chirurgie cardiaque avec mise du patient sous circulation sanguine extracorporelle.

## Revendications

1. Dispositif de délivrance de NO (1) pour fournir un gaz contenant du NO comprenant :
- une ligne d'injection de NO (111) pour acheminer le gaz contenant du NO,
- un dispositif à vanne (113) agencé sur la ligne d'injection (111) pour contrôler la circulation du gaz contenant du NO dans la ligne d'injection (111), ledit dispositif à vanne (113) étant configuré pour être normalement dans une position fermée pour empêcher toute circulation de gaz dans la ligne d'injection (111),
- un dispositif de mesure de débit (112) agencé sur la ligne d'injection (111) pour opérer une ou des mesures de débit du gaz contenant du NO circulant dans la ligne d'injection (111),
- une ligne de secours (201) venant se raccorder fluidiquement à la ligne d'injection (111) en amont et en aval du dispositif à vanne (113), ladite ligne de secours (201) comprenant une électrovanne de secours (202) configurée pour être normalement dans une position ouverte pour permettre une circulation de gaz dans la ligne de secours (201), et un dispositif de contrôle de débit (210) formant ou comprenant un système à orifice calibré proportionnel (204), et
- des moyens de pilotage (130) configurés pour coopérer avec l'électrovanne de secours (202), le dispositif de contrôle de débit (210), le dispositif à vanne (113) et le dispositif de mesure de débit (112),
et dans lequel, en cas de dysfonctionnement engendrant un arrêt de toute coopération avec les moyens de pilotage (130) :
- l'électrovanne de secours (202) est configurée pour passer en position ouverte pour permettre une circulation de gaz dans la ligne de secours (201),
- le dispositif à vanne (113) est configuré pour passer en position fermée pour stopper toute circulation de gaz dans la ligne d'injection (111), et
- le dispositif de contrôle de débit (210) est configuré pour fournir le gaz à un débit gazeux de secours préfixé, où ledit débit gazeux de secours :
o est déterminé par les moyens de pilotage (130) à partir d'au moins une mesure de débit du gaz fournie par le dispositif de mesure de débit (112), pendant un fonctionnement normal du dispositif (1) précédant ledit dysfonctionnement, et
o est préréglé par commande dudit dispositif de contrôle de débit (210) par les moyens de pilotage (130), pendant ledit fonctionnement normal du dispositif (1).

2. Dispositif selon la revendication 1, **caractérisé en ce que** les moyens de pilotage (130) sont en outre configurés pour, pendant le fonctionnement normal du dispositif (1) :
a. commander l'électrovanne de secours (202) pour qu'elle soit en une position fermée empêchant toute circulation de gaz dans la ligne de secours (201),
b. commander le dispositif à vanne (113) pour autoriser une circulation de gaz dans la ligne d'injection (111) et permettre d'opérer au moins une mesure de débit de gaz par le dispositif de mesure de débit (112) et
c. contrôler le dispositif de contrôle de débit (210) pour prérégler le débit gazeux de secours à partir d'au moins une mesure de débit du gaz fournie par le dispositif de mesure de débit (112).

3. Dispositif selon l'une des revendications précédentes, **caractérisé en ce que**, pendant le fonctionnement normal du dispositif (1), le dispositif de mesure de débit (112) est configuré pour opérer plusieurs mesures de débit successives.

4. Dispositif selon la revendication 2, **caractérisé en ce que**, pendant le fonctionnement normal du dispositif (1), les moyens de pilotage (130) sont en outre configurés pour déterminer le débit gazeux de secours à partir d'une ou plusieurs mesures de débit opérées par le dispositif de mesure de débit (112).

5. Dispositif selon la revendication 1, **caractérisé en ce qu'**il comprend des moyens de mémorisation pour mémoriser au moins une partie des mesures de débit successives opérées par le dispositif de mesure de débit (112).

6. Dispositif selon la revendication 4, **caractérisé en ce que** le débit gazeux de secours correspond à la dernière mesure de débit opérée par le dispositif de mesure de débit (112) ayant été opérée avant le dysfonctionnement.

7. Dispositif selon la revendication 1, **caractérisé en ce que** l'électrovanne de secours (202) est du type tout ou rien.

8. Dispositif selon la revendication 1, **caractérisé en ce que** les moyens de pilotage (130) comprennent au moins un microprocesseur.

9. Dispositif selon la revendication 1, **caractérisé en ce qu'**il comprend des moyens d'alimentation électrique pour alimenter au moins les moyens de pilotage (130).

10. Dispositif selon la revendication 1, **caractérisé en ce que** le dispositif de contrôle de débit (210) comprend un moyen actionneur (203) coopérant avec un élément mobile (2041).

11. Dispositif selon la revendication 10, **caractérisé en ce que** le moyen actionneur comprend un moteur électrique alimenté par les moyens d'alimentation électrique.

12. Dispositif selon la revendication 10, **caractérisé en ce que** les moyens de pilotage (130) sont configurés pour :
i) déterminer une ouverture (O) d'orifice calibré (204) correspondant au débit de secours préréglé et
ii) piloter le moyen actionneur (203) pour opérer un déplacement de l'élément mobile (2041) dans une position donnée correspondant audit débit de secours.

13. Dispositif selon la revendication 12, **caractérisé en ce que** les moyens de pilotage (130) sont configurés pour déterminer l'ouverture (O) d'orifice calibré (204) et/ou le débit de secours préréglé à partir d'une table de correspondance mémorisée.

14. Installation de fourniture de gaz (1, 2) à un patient comprenant :
- au moins une source de NO (250) contenant un mélange gazeux NO/N₂,
- un dispositif de délivrance de NO (1) selon l'une des revendications précédentes, alimenté en mélange gazeux NO/N₂ par ladite au moins une source de NO (250),
- une branche inspiratoire (31) d'un circuit patient (3) alimentée en mélange gazeux NO/N₂ par le dispositif de délivrance de NO (1), et
- un ventilateur médical (2) en communication fluidique avec la branche inspiratoire (31) pour alimenter ladite branche inspiratoire (31) en un gaz respiratoire contenant au moins 21% d'oxygène, de préférence de l'air ou un mélange oxygène/azote.

15. Installation selon la revendication 14, **caractérisée en ce que** la source de NO (250) contient un mélange gazeux NO/N₂ contenant entre 100 et 2000 ppmv de NO, le reste étant de l'azote (N₂), conditionné à une pression comprise entre 10 et 250 bar abs.

## Patentansprüche

1. Vorrichtung zur Abgabe von NO (1) zum Bereitstellen eines NO enthaltenden Gases, die umfasst:
- eine NO-Zuführungsleitung (111), um das NO enthaltende Gas zu transportieren,
- eine Ventilvorrichtung (113), die an der Zuführungsleitung (111) angeordnet ist, um die Zirkulation des NO enthaltenden Gases in der Zuführungsleitung (111) zu regeln, wobei die Ventilvorrichtung (113) dazu ausgestaltet ist, normalerweise in einer geschlossenen Stellung zu sein, um jede Zirkulation von Gas in der Zuführungsleitung (111) zu verhindern,
- eine Durchflussmengenmessvorrichtung (112), die an der Zuführungsleitung (111) angeordnet ist, um eine oder mehrere Durchflussmengenmessungen des in der Zuführungsleitung (111) zirkulierenden NO enthaltenden Gases vorzunehmen,
- eine Ersatzleitung (201), die stromauf und stromab der Ventilvorrichtung (113) fluidisch an die Zuführungsleitung (111) angeschlossen ist, wobei die Ersatzleitung (201) ein Ersatzmagnetventil (202) umfasst, das dazu ausgestaltet ist, normalerweise in einer geöffneten Stellung zu sein, um eine Zirkulation von Gas in der Ersatzleitung (201) zu ermöglichen, und eine Durchflussmengenregelungsvorrichtung (210), die ein System mit proportionaler kalibrierter Bohrung (204) bildet oder umfasst, und
- Ansteuerungsmittel (130), die dazu ausgestaltet sind, mit dem Ersatzmagnetventil (202), der Durchflussmengenregelungsvorrichtung (210), der Ventilvorrichtung (113) und der Durchflussmengenmessvorrichtung (112) zusammenzuwirken, und bei der, im Fall einer Funktionsstörung, die zu einem Ausfall jedes Zusammenwirkens mit den Ansteuerungsmitteln (130) führt:
- das Ersatzmagnetventil (202) dazu ausgestaltet ist, in eine geöffnete Stellung überzugehen, um eine Zirkulation von Gas in der Ersatzleitung (201) zu ermöglichen,
- die Ventilvorrichtung (113) dazu ausgestaltet ist, in eine geschlossene Stellung überzugehen, um jede Zirkulation von Gas in der Zuführungsleitung (111) zu stoppen, und
- die Durchflussmengenregelungsvorrichtung (210) dazu ausgestaltet ist, das Gas mit einer vorgegebenen Ersatz-Gasdurchflussmenge bereitzustellen, wobei die Ersatz-Gasdurchflussmenge:
o von den Ansteuerungsmitteln (130) ausgehend von mindestens einer Durchflussmengenmessung des Gases, die von der Durchflussmengenmessvorrichtung (112) bereitgestellt wird, während eines Normalbetriebs der Vorrichtung (1), der der Funktionsstörung vorausgegangen ist, bestimmt wird, und
o durch Steuern der Durchflussmengenregelungsvorrichtung (210) durch die Ansteuerungsmittel (130) während des Normalbetriebs der Vorrichtung (1) voreingestellt wird.

2. Vorrichtung nach Anspruch 1, **dadurch gekennzeichnet, dass** die Ansteuerungsmittel (130) ferner dazu ausgestaltet sind, während des Normalbetriebs der Vorrichtung (1):
a. das Ersatzmagnetventil (202) zu steuern, damit es in einer geschlossenen Stellung ist, die jede Zirkulation von Gas in der Ersatzleitung (201) verhindert,
b. die Ventilvorrichtung (113) zu steuern, um eine Zirkulation von Gas in der Zuführungsleitung (111) zuzulassen und zu ermöglichen, dass mindestens eine Gasdurchflussmengenmessung von der Durchflussmengenmessvorrichtung (112) vorgenommen wird, und
c. die Durchflussmengenregelungsvorrichtung (210) zu regeln, um die Ersatz-Gasdurchflussmenge ausgehend von mindestens einer Durchflussmengenmessung des Gases voreinzustellen, die von der Durchflussmengenmessvorrichtung (112) bereitgestellt wird.

3. Vorrichtung nach einem der vorhergehenden Ansprüche, **dadurch gekennzeichnet, dass**, während des Normalbetriebs der Vorrichtung (1), die Durchflussmengenmessvorrichtung (112) dazu ausgestaltet ist, mehrere aufeinander folgende Durchflussmengenmessungen vorzunehmen.

4. Vorrichtung nach Anspruch 2, **dadurch gekennzeichnet, dass**, während des Normalbetriebs der Vorrichtung (1), die Ansteuerungsmittel (130) ferner dazu ausgestaltet sind, die Ersatz-Gasdurchflussmenge ausgehend von einer oder mehreren Durchflussmengenmessungen zu bestimmen, die von der Durchflussmengenmessvorrichtung (112) vorgenommen werden.

5. Vorrichtung nach Anspruch 1, **dadurch gekennzeichnet, dass** sie Speichermittel umfasst, um mindestens einen Teil der aufeinander folgenden Durchflussmengenmessungen zu speichern, die von der Durchflussmengenmessvorrichtung (112) vorgenommen werden.

6. Vorrichtung nach Anspruch 4, **dadurch gekennzeichnet, dass** die Ersatz-Gasdurchflussmenge der letzten von der Durchflussmengenmessvorrichtung (112) vorgenommenen Durchflussmengenmessung entspricht, die vor der Funktionsstörung vorgenommen worden ist.

7. Vorrichtung nach Anspruch 1, **dadurch gekennzeichnet, dass** das Ersatz-Magnetventil (202) vom Auf/Zu-Typ ist.

8. Vorrichtung nach Anspruch 1, **dadurch gekennzeichnet, dass** die Ansteuerungsmittel (130) mindestens einen Mikroprozessor umfassen.

9. Vorrichtung nach Anspruch 1, **dadurch gekennzeichnet, dass** sie Stromversorgungsmittel umfasst, um mindestens die Ansteuerungsmittel (130) zu versorgen.

10. Vorrichtung nach Anspruch 1, **dadurch gekennzeichnet, dass** die Durchflussmengenregelungsvorrichtung (210) ein Aktormittel (203) umfasst, das mit einem mobilen Element (2041) zusammenwirkt.

11. Vorrichtung nach Anspruch 10, **dadurch gekennzeichnet, dass** das Aktormittel einen Elektromotor umfasst, der von den Stromversorgungsmitteln versorgt wird.

12. Vorrichtung nach Anspruch 10, **dadurch gekennzeichnet, dass** die Ansteuerungsmittel (130) dazu ausgestaltet sind:
i) eine Öffnung (O) der kalibrierten Bohrung (204) zu bestimmen, die der voreingestellten Ersatzdurchflussmenge entspricht, und
ii) das Aktormittel (203) anzusteuern, um eine Verlagerung des mobilen Elements (2041) in eine gegebene Position vorzunehmen, die der Ersatzdurchflussmenge entspricht.

13. Vorrichtung nach Anspruch 12, **dadurch gekennzeichnet, dass** die Ansteuerungsmittel (130) dazu ausgestaltet sind, die Öffnung (O) der kalibrierten Bohrung (204) und/oder die voreingestellte Ersatzdurchflussmenge ausgehend von einer gespeicherten Korrespondenztabelle zu bestimmen.

14. Anlage zur Bereitstellung von Gas (1,2) für einen Patienten, umfassend:
- mindestens eine NO-Quelle (250), die ein NO/N₂-Gasgemisch enthält,
- eine Vorrichtung zur Abgabe von NO (1) nach einem der vorhergehenden Ansprüche, die von der mindestens einen NO-Quelle (250) mit NO/N₂-Gasgemisch versorgt wird,
- einen Einatemzweig (31) eines Patientenkreislaufs (3), der von der Vorrichtung zur Abgabe von NO (1) mit NO/N₂-Gasgemisch versorgt wird, und
- ein medizinisches Beatmungsgerät (2) in Fluidverbindung mit dem Einatemzweig (31), um den Einatemzweig (31) mit einem Atemgas zu versorgen, das mindestens 21 % Sauerstoff, bevorzugt Luft oder ein Sauerstoff/Stickstoff-Gemisch enthält.

15. Anlage nach Anspruch 14, **dadurch gekennzeichnet, dass** die NO-Quelle (250) ein NO/N₂-Gasgemisch enthält, das zwischen 100 und 2000 ppmv NO enthält, wobei der Rest Stickstoff (N₂) ist, das auf einen Druck zwischen 10 und 250 bar abs konditioniert ist.

## Claims

1. NO delivery device (1) for supplying an NO-containing gas, comprising:
- an NO injection line (111) for conveying the NO-containing gas,
- a valve device (113) arranged on the injection line (111) in order to control the circulation of the NO-containing gas in the injection line (111), said valve device (113) being configured to be normally in a closed position for preventing any circulation of gas in the injection line (111),
- a flow rate measurement device (112) arranged on the injection line (111) in order to perform one or more measurements of the flow rate of the NO-containing gas circulating in the injection line (111),
- an emergency line (201) which connects fluidically to the injection line (111) upstream and downstream of the valve device (113), said emergency line (201) comprising an emergency solenoid valve (202) configured to be normally in an open position in order to permit a circulation of gas in the emergency line (201), and a flow rate control device (210) forming or comprising a system with a proportional calibrated orifice (204), and
- operating means (130) configured to cooperate with the emergency solenoid valve (202), the flow rate control device (210), the valve device (113) and the flow rate measurement device (112),
and in which, in the event of a malfunction causing shutdown of all cooperation with the operating means (130):
- the emergency solenoid valve (202) is configured to pass to an open position in order to permit a circulation of gas in the emergency line (201),
- the valve device (113) is configured to pass to a closed position in order to stop all circulation of gas in the injection line (111), and
- the flow rate control device (210) is configured to supply the gas at a pre-fixed emergency flow rate of gas, where said emergency flow rate of gas:
∘ is determined by the operating means (130) on the basis of at least one gas flow rate measurement supplied by the flow rate measurement device (112), during a normal functioning of the device (1) prior to said malfunction, and
∘ is pre-regulated through command of said flow rate control device (210) by the operating means (130), during said normal functioning of the device (1).

2. Device according to Claim 1, **characterized in that** the operating means (130) are further configured in order, during the normal functioning of the device (1), to:
a. command the emergency solenoid valve (202) such that the latter is in a closed position preventing all circulation of gas in the emergency line (201),
b. command the valve device (113) to enable a circulation of gas in the injection line (111) and to permit at least one measurement of gas flow rate to be carried out by the flow rate measurement device (112), and
c. control the flow rate control device (210) in order to pre-regulate the emergency flow rate of gas on the basis of at least one gas flow rate measurement supplied by the flow rate measurement device (112).

3. Device according to one of the preceding claims, **characterized in that**, during the normal functioning of the device (1), the flow rate measurement device (112) is configured to perform several successive flow rate measurements.

4. Device according to Claim 2, **characterized in that**, during the normal functioning of the device (1), the operating means (130) are further configured to determine the emergency flow rate of gas on the basis of one or more flow rate measurements performed by the flow rate measurement device (112).

5. Device according to Claim 1, **characterized in that** it comprises storage means for storing at least some of the successive flow rate measurements performed by the flow rate measurement device (112).

6. Device according to Claim 4, **characterized in that** the emergency flow rate of gas corresponds to the last flow rate measurement performed by the flow rate measurement device (112) that was performed prior to the malfunction.

7. Device according to Claim 1, **characterized in that** the emergency solenoid valve (202) is of the all-or-nothing type.

8. Device according to Claim 1, **characterized in that** the operating means (130) comprise at least one microprocessor.

9. Device according to Claim 1, **characterized in that** it comprises electrical power supply means for supplying at least the operating means (130).

10. Device according to Claim 1, **characterized in that** the flow rate control device (210) comprises an actuator means (203) cooperating with a movable element (2041).

11. Device according to Claim 10, **characterized in that** the actuator means comprises an electric motor powered by the electrical power supply means.

12. Device according to Claim 10, **characterized in that** the operating means (130) are configured to:
i) determine an opening (O) of a calibrated orifice (204) corresponding to the pre-regulated emergency flow rate, and
ii) operate the actuator means (203) in order to cause a displacement of the movable element (2041) to a given position corresponding to said emergency flow rate.

13. Device according to Claim 12, **characterized in that** the operating means (130) are configured to determine the opening (O) of the calibrated orifice (204) and/or the pre-regulated emergency flow rate on the basis of a stored lookup table.

14. Installation (1, 2) for supplying gas to a patient, comprising:
- at least one NO source (250) containing a gaseous mixture NO/N₂,
- an NO delivery device (1) according to one of the preceding claims, supplied with a gaseous mixture NO/N₂ by said at least one NO source (250),
- an inhalation branch (31) of a patient circuit (3) supplied with a gaseous mixture NO/N₂ by the NO delivery device (1), and
- a medical ventilator (2) in fluidic communication with the inhalation branch (31) in order to supply said inhalation branch (31) with a respiratory gas containing at least 210 of oxygen, preferably air or an oxygen/nitrogen mixture.

15. Installation according to Claim 14, **characterized in that** the NO source (250) contains a gaseous mixture NO/N₂ containing between 100 and 2000 ppmv of NO, the remainder being nitrogen (N₂), stored at a pressure of between 10 and 250 bar abs.
